# EUROPEAN PATENT APPLICATION

(11) **EP 3 382 376 A1**
(43) Date of publication of application: **03.10.2018**
(21) Application number: 17162985.0
(22) Date of filing: 27.03.2017
(51) Int. Cl.: G01N 21/25, G01N 35/00, G01N 33/49, H04N 5/225, G06K 7/00, G01N 35/02

(54) **METHOD FOR DETERMINING COLOR INFORMATION AND LABORATORY AUTOMATION SYSTEM**

(71) Applicant: Roche Diagnostics GmbH, 68305 Mannheim (DE); F. Hoffmann-La Roche AG, 4070 Basel (CH)
(72) Inventor: GARCIA BUSTOS, Soraya, 70199 Stuttgart (DE); REIN, Michael, 70736 Fellbach (DE)
(74) Representative: Patentanwälte Ruff, Wilhelm, Beier, Dauster & Partner mbB

(57) **Abstract**

The invention relates to a method for determining color information of an object, e.g. sample container (145) or sample (148), of a laboratory automation system (10) using a gray scale camera (160) and at least two different colors for illumination, e.g. with light emiting diodes (164, 165). The invention relates further to a laboratory automation system (10) being adapted to perform such a method.

## Description

### Applicable field and prior art

The invention relates to a method for determining color information and to a laboratory automation system.

WO 2013/064656 A1 discloses a laboratory automation system. It has been found that during operation of such laboratory automation systems samples or sample containers have to be identified in a plurality of instances. Such identification can, for example, be performed using cameras being adapted to take an image of an object such that its color or other information can be extracted from that image. However, cameras being able to differentiate certain colors are expensive and/or slow regarding the image capturing capabilities. Further, digital color cameras having a RGB filter arranged before the image sensor provide a lower effective resolution compared to digital grey scale cameras. If e.g. a digital camera is used for barcode reading, a digital color camera needs a four times higher resolution than a digital grey scale camera to provide equal results.

### Object and solution

It is thus an object of the invention to provide for a method for determining color information of an object of a laboratory automation system having lower equipment requirements. It is a further object of the invention to provide for a laboratory automation system being adapted to perform such a method.

This object is achieved by a method according to claim 1 and by a laboratory automation system according to claim 9. Preferred embodiments can, for example, be derived from the respective dependent claims.

The invention relates to a method for determining color information of an object of a laboratory automation system. The method comprises the following steps:
- providing a digital gray scale camera such that the digital gray scale camera views the object or at least a subarea of the object,
- illuminating the object or subarea with light having a first color, wherein the emitted spectrum of light having the first color is typically known,
- generating first picture data by taking a first picture of the object or subarea while the object or subarea is illuminated with the light having the first color using the digital gray scale camera,
- illuminating the object or subarea with light having a second color being different from the first color, wherein the emitted spectrum of light having the second color is typically known,
- generating second picture data by taking a second picture of the object or subarea while the object or subarea is illuminated with the light having the second color using the digital gray scale camera, and
- calculating the color information using the first picture data and the second picture data.

The calculation of the color information using the first picture data and the second picture data may be performed using only specific sections within the first picture data and the second picture data, wherein the specific section within the first picture data and the specific section within the second picture data correspond to one another, i.e. correspond to identical parts of the object, e.g. a cap of a sample container. The specific sections may be determined by image processing.

The calculation of the color information is based on the fact that the absorption of specific wavelengths comprised in the light used for illumination is dependent on the color of the object.

If e.g. the object is red, the absorption of the wavelengths corresponding to the red color is smaller than the absorption of the wavelengths corresponding to non-red colors. The gray scale camera may take the first picture of the red object while the object or subarea is illuminated with wavelengths corresponding to the red color. Then, the gray scale camera may take the second picture of the red object while the object or subarea is illuminated with wavelengths corresponding to non-red colors, e.g. the green or blue color. The first picture data differs from the second picture data due to the wavelength specific absorption of the red object. Based on the absorption caused differences in both picture data the object is then calculated to have a higher red color content compared to the other illuminating colors. In other words, a simple color spectroscope may be embodied by the inventive method.

Calculating the color information using the first picture data and the second picture data may comprise the sub steps: comparing the first picture data and the second picture data, and calculating the color information based on the comparison.

Reference is also made to the relevant technical literature regarding the absorption of specific wavelengths of illuminated colored objects.

The color information can be indicative of a color of the object or of a color of the subarea of the object. For example, it can be determined if the subarea has a red, green, blue, yellow, purple, white or black color. Such color information can give rise to a specific treatment of such an object or can yield other information that is important for operation of the laboratory automation system.

It should be noted that not only one color can be identified, but also a plurality of colors in a certain pattern, for example a color barcode, which may be analyzed in a plurality of pixels.

By means of the inventive method, a gray scale camera may be used to gain color information of an object. It is not necessary to use expensive and/or slow color-enabled cameras. Generating different colors for illumination is cost effective, as light emitting diodes having different colors are cheaply available. Alternatively or additionally, filters can be used in order to get different colors out of one light source.

According to an embodiment, the method further comprises a step of illuminating the subarea with light having a third color being different from the first color and being different from the second color. It further comprises a step of generating third picture data by taking a third picture of the subarea while the subarea is illuminated with the light having the third color using the digital gray scale camera, and further comprises calculating the color information using the first picture data, the second picture data and the third picture data. Thus, reliability can be increased, as a further color is used in order to obtain the color information.

According to an embodiment, the method further comprises a plurality of steps of illuminating the subarea with a respective light having a respective color such that all colors are mutually different from each other. It further comprises generating a plurality of picture data by taking respective pictures of the subarea while the subarea is illuminated with the light having a respective color using the digital gray scale camera, and calculating the color information using all picture data. By means of such an embodiment, a plurality of colors can be used in order to further increase reliability of the method, and especially of the obtained color information. For example, four, five, six, seven or eight different colors can be used. It should be noted that any other number of colors can be used for performing the method.

According to an embodiment, the object is a sample container, a sample contained in the sample container, or a cap covering the sample container. Such objects have to be identified in typical operations of laboratory automation systems, wherein color information can typically be used for identification tasks.

According to an embodiment, the light having the first color and/or the light having the second color, and/or any other light having a respective color, for example the third color, is/are selected in the visible range or in the infrared range. Such visible range can, for example, be light having a wavelength between 380 nm and 780 nm.

According to an embodiment, the first color and/or the second color and/or the third color or any other color is/are selected from a group consisting of red, green and blue. Such colors can be easily obtained, for example by using filters and a white-light source or light emitting diodes, and have been proven suitable for typical color recognition operations.

According to an embodiment, the method further comprises the step of determining sample related and/or sample container related information using the color information. Such sample related and/or sample container related information can, for example, be information relating to the type of sample or operations having to be performed with the sample.

According to an embodiment, the method further comprises the step of assigning the sample to a class out of a predetermined set of classes using the color information. The sample may be a blood sample or a blood plasma sample. The predetermined set of classes comprises a good class, a lipemic class, a hemolytic class, an icteric class, and an error class. The lipemic class, hemolytic class, icteric class and good class may be typical classes which can be assigned to a blood plasma sample. The good class may contain those samples which are not assigned to the class lipemic, hemolytic or icteric. The error class denotes samples having non-plausible color information (values).

When the sample is assigned to the lipemic class, it may be a lipemic sample which has an elevated level of lipids. This may, for example, be an indication of a disorder of the fat metabolism. The first color or the second color may comprise a lipemic wavelength in the range of 610 nm to 700 nm, preferably 650 nm or 685 nm. The blood plasma sample may be assigned to the lipemic class when the calculated color information at the lipemic wavelength is less than a lipemic threshold value. Therefore, the lipemic sample may be detected and assigned to the lipemic class by calculating the color information using the first picture data and the second picture data.

When the sample is assigned to the hemolytic class, it may be a hemolytic sample which has an elevated level of hemoglobin. This may, for example, be an indication of particular anemias, transfusion reactions or malaria. The first color or the second color may comprise a hemolytic wavelength in the range of from 500 nm to 600 nm, preferably 541 nm. The blood plasma sample may be assigned to the hemolytic class when the calculated color information at the hemolytic wavelength is less than a hemolytic threshold value and if the sample is not assigned to the lipemic class. Therefore, the hemolytic sample may be detected and assigned to the hemolytic class by calculating the color information using the first picture data and the second picture data.

When the blood plasma sample is assigned to the icteric class, it may be an icteric sample which has an elevated level of bilirubin. This may, for example, be an indication of a disease of the liver. The first color or the second color may comprise an icteric wavelength in the range of from 450 nm to 485 nm, preferably 470 nm. The blood plasma sample may be assigned to the icteric class when the calculated color information at the icteric wavelength is less than an icteric threshold value and if the sample is not assigned to the lipemic class or to the hemolytic class. Therefore, the icteric sample may be detected and assigned to the icteric class by calculating the color information using the first picture data and the second picture data.

The invention further relates to a laboratory automation system being adapted to perform the method according to the invention.

The laboratory automation system comprises a digital gray scale camera. The laboratory automation system further comprises illumination means for generating the light having the first color and generating the light having the second color. It may further comprise illumination means for generating the light having the third color or illumination means for generating the light having any further color.

The laboratory automation system further comprises a control device, wherein the control device is configured to control the digital gray scale camera and the illuminating means such that the method according to the invention is performed.

By means of the inventive laboratory automation system, the inventive method can be advantageously used in order to get information about objects used by the laboratory automation system, like sample containers or sample container carriers. Usage of expensive color-enabled cameras can be omitted.

According to an embodiment, the illumination means comprises a first light emitting diode emitting light having a wavelength corresponding to the first color and comprises a second light emitting diode emitting light having a wavelength corresponding to the second color. It can also comprise a third light emitting diode emitting light having a wavelength corresponding to the third color, and it can further comprise a plurality of further light emitting diodes emitting light having respective wavelengths corresponding to any further color. Using such light emitting diodes has been proven a cheap and reliable way to generate light having specific colors.

According to an embodiment, the illumination means comprises a light source, especially for emitting light having a broad wave length spectrum, and a filter being adapted to filter light emitted by the light source such that the light having the first color or the light having the second color or the light having the third color or any further color is generated. Using such filters has also been proven to be a cheap and reliable way to generate different colors.

The filter may be an acryl glass filter.

According to an embodiment, the laboratory automation system comprises a number (e.g. 1 to 100) of laboratory stations. The number of laboratory stations may comprise pre-analytical, analytical and/or post-analytical stations.

It should be noted that processing of samples in such laboratory stations can be influenced by the color information detected by the inventive method.

Pre-analytical stations may be adapted to perform any kind of pre-processing of samples, sample containers and/or sample container carriers.

Analytical stations may be adapted to use a sample or part of the sample and a reagent to generate a measuring signal, the measuring signal indicating if and in which concentration, if any, an analyte is existing.

Post-analytical stations may be adapted to perform any kind of post-processing of samples, sample containers and/or sample container carriers.

The pre-analytical, analytical and/or post-analytical stations may comprise at least one of a decapping station, a recapping station, an aliquot station, a centrifugation station, an archiving station, a pipetting station, a sorting station, a tube type identification station, a sample quality determining station, an add-on buffer station, a liquid level detection station, and a sealing/desealing station.

### Short description of the drawing

The invention will be described in detail with respect to the drawings schematically depicting embodiments of the invention. In detail:
- Fig. 1: shows a laboratory automation system being adapted to perform the inventive method, and
- Fig. 2: shows a table of picture data.

### Detailed description of the embodiment

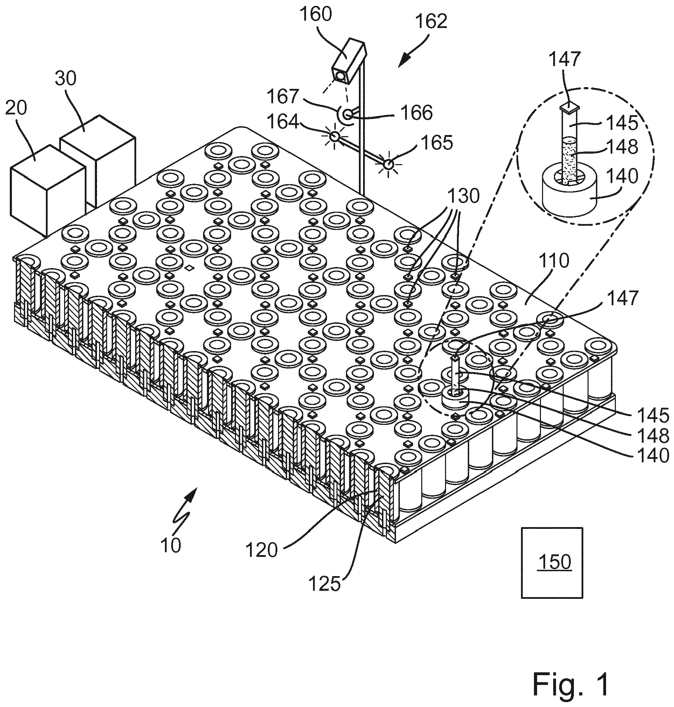

Fig. 1 shows a laboratory automation system 10 comprising a number of pre-analytical, analytical and/or post-analytical stations 20, 30. Self-evidently, more than the two stations 20, 30 depicted in fig. 1 may be comprised in the laboratory automation system 10.

The laboratory automation system 10 comprises a transport plane 110, under which a plurality of electro-magnetic actuators in the form of electromagnets 120 are positioned. The electromagnets 120 are implemented as solenoids each having a solid ferromagnetic core 125.

Sample container carriers 140 are positioned on the transport plane 110 and can be moved by means of the electromagnets 120, because each sample container carrier 140 has an internal permanent magnet. While it should be understood that a plurality of sample container carriers 140 can be positioned on the transport plane 110, due to simplicity only one sample container carrier 140 is depicted in fig. 1. The sample container carrier 140 holds a sample container 145, in which a sample 148 to be analyzed can be contained. The sample container 145 is covered by means of a cap 147, being embodied as a foil having a specific color.

The laboratory automation system 10 is adapted to transport the sample container carriers 140 and/or the sample containers 145 between the laboratory stations 20, 30. The laboratory stations 20, 30 are positioned adjacent to the transport plane 110 such that a sample container carrier 140 can be used to transport a sample contained in the sample container 145 to a respective laboratory station 20, 30.

A plurality of Hall-sensors 130 is arranged such that positions of respective sample container carriers 140 on the transport surface 110 can be detected.

The laboratory automation system 10 further comprises a control device 150. The control device 150 is configured to control movement of the sample container carriers 140 on the transport plane 110 by driving the electromagnets 120 such that the sample container carriers 140 independently and simultaneously move along corresponding transport paths.

The laboratory automation system further comprises a gray scale camera 160 and illumination means 162.

The illumination means 162 comprises two light emitting diodes 164, 165. The two light emitting diodes 164, 165 are positioned adjacent to the gray scale camera 160 and can deliver the colors of red and green, respectively. They illuminate a part of the transport plane 110 of which a picture can be taken by the gray scale camera 160. If an object, for example a sample container carrier 140, a sample container 145 and/or a sample 148, is to be identified, the object is moved over the transport plane 110 such that it is in the field of vision of the gray scale camera 160. Then, the two light emitting diodes 164, 165 are turned on separately and successively. Thus, the object is successively illuminated by red and green light. During respective illumination by each color, a respective picture is taken by the gray scale camera 160. The pictures are transferred from the camera 160 to the control device 150, which is adapted to calculate color information using these pictures.

Such color information can, for example, be indicative of a color of a sample 148 contained in a sample container 145 or indicative of a color of a foil with which the sample container 145 is capped. Such color information can further yield information about contained samples 148 or tasks to be done with such a sample, and can thus influence how the sample 148 is being processed, for example using the laboratory stations 20, 30.

For illustrative purposes, also a different way of color-specific illumination is shown in fig. 1, namely using a white spectrum light source 166, being embodied as a white light emitting diode or a light bulb, and a color filter 167 as part of the illumination means 162. The color filter 167 has a certain narrow wavelength spectrum in which it transmits light emitter from the light source 166. It should be noted that the method can also be performed such that a plurality of filters transmitting different colors are provided and are exchangeable during operation.

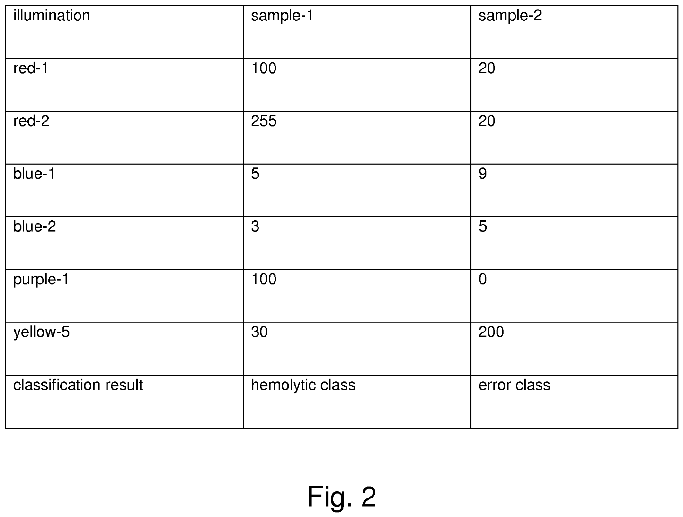

Fig. 2 shows a schematic table of picture data values in the range of 0 to 255 (8 bit values). Two samples sample-1 and sample-2 are sequentially illuminated with six different colors named as red-1, red-2, blue-1, blue-2, purple-1, yellow-5 generated by six different laser-diodes. The resulting picture data values of a representative pixel generated by the greyscale camera are shown in the second and third column, respectively.

Based on the corresponding picture data values, sample-1 is assigned to the hemolytic class and sample-2 is assigned to an error class, since the color information values are not plausible.

## Claims

1. Method for determining color information of an object (145, 147, 148) of a laboratory automation system (10), the method comprising the steps:
- providing a digital greyscale camera (160) such that the digital grayscale camera (160) views at least a subarea of the object (145, 147, 148),
- illuminating the subarea with light having a first color,
- generating first picture data by taking a first picture of the subarea while the subarea is illuminated with the light having the first color using the digital grayscale camera (160),
- illuminating the subarea with light having a second color being different from the first color,
- generating second picture data by taking a second picture of the subarea while the subarea is illuminated with the light having the second color using the digital grayscale camera (160), and
- calculating the color information using the first picture data and the second picture data.

2. Method according to claim 1, **characterized in that**
- the method further comprises the step of illuminating the subarea with light having a third color being different from the first color and being different from the second color,
- generating third picture data by taking a third picture of the subarea while the subarea is illuminated with the light having the third color using the digital grayscale camera (160), and
- calculating the color information using the first picture data, the second picture data and the third picture data.

3. Method according to claim 1 or 2, **characterized in that**
- the method further comprises a plurality of steps of illuminating the subarea with a respective light having a respective color such that all colors are mutually different from each other,
- generating a plurality of picture data by taking respective pictures of the subarea while the subarea is illuminated with the light having a respective color using the digital grayscale camera (160), and
- calculating the color information using all picture data.

4. Method according to one of the preceding claims, **characterized in that**
- the object is a sample container (145), a sample (148) contained in the sample container (145) or a cap (147) covering the sample container (145).

5. Method according to one of the preceding claims, **characterized in that**
- the light having the first color and/or the light having the second color is/are selected in the visible range or in the infrared range.

6. Method according to one of the preceding claims, **characterized in that**
- the first color and/or the second color is/are selected from a group consisting of red, green and blue.

7. Method according to one of the preceding claims, **characterized in that**
- the method further comprises the step of determining sample related and/or sample container related information using the color information.

8. Method according to claim 7, **characterized in that**
- the method further comprises the step of assigning the sample to a class out of a predetermined set of classes using the color information,
- wherein the predetermined set of classes comprises a good class, a lipemic class, a hemolytic class, an icteric class, and an error class.

9. Laboratory automation system (10) being adapted to perform the method of one of the preceding claims, comprising
- the digital grayscale camera (160),
- illumination means (162) for generating the light having the first color and generating the light having the second color, and
- a control device (150), wherein the control device (150) is configured to control the digital grayscale camera (160) and the illumination means (162) such that the method according to one of the preceding claims is performed.

10. Laboratory automation system (10) according to claim 9, **characterized in that**
- the illumination means (162) comprise a first light emitting diode (164) emitting light having a wavelength corresponding to the first color and comprise a second light emitting diode (165) emitting light having a wavelength corresponding to the second color.

11. Laboratory automation system (10) according to claim 9 or 10, **characterized in that**
- the illumination means (162) comprise a light source (166) and a filter (167) being adapted to filter light emitted by the light source (166) such that the light having the first color or the light having the second color is generated.

12. Laboratory automation system (10) according to claim 11, **characterized in that**
- the filter (167) is an acryl glass filter.

13. Laboratory automation system (10) according to one of claims 9 to 12, comprising
- a number of laboratory stations (20, 30).

14. Laboratory automation system (10) according to claim 13, **characterized in that**
- the number of laboratory stations (20, 30) comprise pre-analytical, analytical and/or post-analytical stations.
